# FASCICULE DE BREVET EUROPEEN

(11) **EP 2 680 900 B1**
(45) Date de publication et mention de la délivrance du brevet: **29.08.2018**
(21) Numéro de dépôt: 12718351.5
(22) Date de dépôt: 02.03.2012
(51) Int. Cl.: A61M 25/06, A61M 5/158

(54) **INSERTEUR DE CANULE**
KANÜLENEINFÜHRER
CANNULA INSERTER

(30) Priorité: 03.03.2011 EP 11156889
(43) Date de publication de la demande: 08.01.2014
(73) Titulaire: Debiotech S.A., 1004 Lausanne (CH)
(72) Inventeur: DA ROS, Jérôme, 74200 Thonon les Bains (FR); NEFTEL, Frédéric, 1005 Lausanne (CH)
(74) Mandataire: Weihs, Bruno Konrad
(86) Numéro de dépôt international: PCT/IB2012/050994
(87) Numéro de publication internationale: WO 2012/117379

(56) Documents cités:
- EP-A1- 1 970 083
- EP-A1- 1 970 084
- EP-A1- 1 970 091
- FR-A1- 2 752 164
- US-A1- 2005 101 912

## Description

### Domaine de l'invention

L'invention concerne l'insertion transdermique et la fixation d'une canule préalablement à l'administration et/ou à l'aspiration d'un liquide à travers la canule.

### Etat de la technique

Un inserteur de canule tel que décrit dans EP 1 743 667 A2 ou US 6,607,509 B2 comprend un corps sensiblement cylindrique à l'intérieur duquel se meut un piston. En se déplaçant vers l'extrémité distale de l'inserteur, le piston entraîne la canule ainsi qu'une aiguille - agissant comme mandrin - qui est disposée à l'intérieur de la canule. Une fois la canule mise en place, l'aiguille est retirée.

L'insertion de la canule et le retrait de l'aiguille constituent deux actions importantes dans le fonctionnement d'un inserteur de canule.

Dans les inserteurs de l'état de la technique, ces deux actions sont réalisées indépendamment l'une de l'autre. L'insertion peut être manuelle alors que le retrait de l'aiguille est automatique ou vice-versa.

EP1970091 décrit une tête d'insertion dans lequel le dispositif de perforation contenue dans un boîtier est automatiquement pivoté, après son retrait de la canule, vers une position protégée dans le boîtier en pressant sur l'une des parties du boîtier.

A ce jour il n'existe pas de système suffisamment économique permettant de combiner ces deux actions, en particulier lorsque l'on souhaite insérer la canule à une vitesse relativement importante, ce qui offrirait l'avantage de simplifier l'ensemble, de diminuer le temps d'intervention et d'augmenter la sécurité. En outre, les inserteurs de l'état de la technique ne permettent pas d'insérer une canule et de la fixer aisément sur un patch et/ou de réaliser une telle fonction à l'aide d'un dispositif suffisamment économique pour être conçu comme usage unique.

Il est donc hautement souhaitable d'avoir un inserteur de canule remédiant aux inconvénients précités.

### Description générale de l'invention

Un des buts de la présente invention est d'offrir la possibilité à l'utilisateur de réaliser la double opération « insertion de l'ensemble canule-aiguille » et « protection de l'aiguille » au moyen d'un même élément déclencheur, de préférence à usage unique. L'invention permet de simplifier l'utilisation de l'inserteur par le patient en minimisant le nombre de manipulations. Ce qui apporte les avantages suivants : diminution du risque d'erreurs et réduction de la taille du dispositif.

Ce but est obtenu grâce à l'inserteur faisant l'objet des revendications ci-jointes.

Inserteur de canule comprenant un boîtier ayant une extrémité distale destinée à venir à proximité de la peau d'un patient et une extrémité proximale opposée, un piston fixé au moins temporairement par des moyens de retenue, des moyens d'entrainement agencés de manière à déplacer le piston en direction opposée au déclencheur, des moyens de libération destinés à libérer le piston ; l'inserteur comprenant en outre une aiguille fixée à un support ; le piston étant monté dans le boîtier de manière à ce qu'une fois libéré, il atteigne et reste temporairement dans une première position, puis une deuxième position; l'aiguille et son support étant en outre disposés de manière à pouvoir être entraînés par le piston selon une direction correspondant à l'orientation principale du boîtier, avant que le piston atteigne ladite première position, et de manière à pouvoir effectuer une rotation automatique autour d'un axe perpendiculaire à ladite orientation principale lors de la transition du piston entre ladite première et ladite deuxième position.

Dans le présent fascicule, le terme « canule » désigne un tube destiné à être au moins en partie introduit dans le corps du patient afin de permettre le passage de liquides, en particulier de l'insuline pour les patients diabétiques.

Les moyens d'entrainement peuvent être constitués d'un ressort ou d'un élastique ou de lames élastiques.

Les moyens d'entrainement peuvent être composés d'une unique source d'énergie.

De préférence, les moyens d'entrainement sont des moyens de propulsion disposés entre le piston et le déclencheur. Dans une autre réalisation, les moyens d'entrainement peuvent être des moyens de traction disposés entre le piston et l'extrémité distale de l'inserteur.

L'inserteur est de préférence prêt à l'emploi, ainsi les moyens de propulsion peuvent être pré-chargés et les éléments d'insertion en position vertical logés dans le boitier.

Des éléments de sécurité peuvent être disposés soit sur l'extrémité proximale soit sur l'extrémité distale de l'inserteur. Ces éléments de sécurité peuvent se présenter sous la forme de couvercles ou d'opercules à retirer avant l'utilisation de l'inserteur.

Selon un mode de réalisation, l'extrémité distale des moyens de propulsion fait office de piston.
Exprimé différemment, les moyens de propulsion et le piston peuvent faire partie de la même pièce, en particulier dans une réalisation comprenant des lames élastiques comme moyens de propulsion.

Les moyens de retenue du piston peuvent être disposés en n'importe quel endroit permettant d'obtenir l'effet recherché. De préférence, ils sont disposés sur le déclencheur. Lesdits moyens de retenue peuvent retenir soit le piston, p.ex. à son extrémité distale, soit le support de l'aiguille.

Les moyens de libération du piston peuvent être disposés en n'importe quel endroit permettant d'obtenir l'effet recherché. De préférence, ils sont disposés sur la paroi interne du boîtier.

Le déclencheur peut se présenter de diverses manières, p.ex. sous forme d'un ou plusieurs boutons activables par l'utilisateur. Ces boutons peuvent être positionnés dans l'axe ou perpendiculaire à l'inserteur.

De préférence, l'inserteur comprend un élément de sécurité qui empêche toute activation accidentelle du déclencheur. L'élément de sécurité peut se présenter sous la forme d'un ou deux boutons disposés sur la face latérale du boîtier. En appuyant sur le ou les boutons, le déclencheur n'est plus retenu de sorte que son activation est possible. Dans une autre réalisation, les éléments de sécurité sont combinés avec le déclencheur, de sorte qu'en appuyant simultanément sur le ou les éléments de sécurité et le déclencheur, le déclenchement soit effectif.

Le déclencheur et les boutons de sécurité sont de préférence disposés de sorte que l'inserteur soit utilisable avec une seule main.

Alternativement, le déclencheur est libéré par déformation du boîtier.

La canule et son support peuvent être déplacés de différentes manières. Avantageusement, le piston comporte une face distale destinée à exercer exclusivement un effet de poussée sur la canule et son support. Dans cette variante, il n'existe donc pas de moyens de retenue (p.ex. des clips) qui fixeraient la canule et son support au piston.
Toujours dans le cadre de cette variante, la canule et son support sont fixés seulement à l'aiguille. Ce mode de fixation peut être obtenu par friction, entre l'aiguille et un septum disposé dans la canule.

L'insertion de la canule se fait par une action, à savoir une pression sur le déclencheur avec éventuellement, comme indiqué précédemment, le déclenchement préalable d'une sécurité qui retient le déclencheur. La rétraction (rotation) de l'aiguille usagée se fait de préférence automatiquement après son retrait de la canule.
L'inserteur est retiré manuellement. Alors que et/ou une fois que le retrait ait été effectué, les moyens de propulsion déplacent le piston dans l'orientation principale vers sa deuxième position, ce qui a pour effet d'entrainer un mouvement rotatif de l'aiguille.

Lors du retrait, la canule reste en place dans le patient grâce à des moyens de retenue disposés sur son support et un patch préalablement installé sur la peau du patient ou grâce à un adhésif solidaire au support de canule.

De préférence, le boîtier comporte un espace de protection dans lequel se loge l'aiguille rétractée et des moyens de rétention qui évite toute rotation inverse de ladite aiguille. De préférence, ces moyens de rétention sont assurés par la force résiduelle des moyens de propulsion.

Principalement, l'inserteur selon l'invention comprend les éléments suivants : un boîtier, un déclencheur, un piston, des moyens d'entrainement et une aiguille, cette dernière étant de préférence surmoulée dans un élément plastique, la partie proximale de cet élément constituant le support d'aiguille.
Le support d'aiguille est lié au piston de préférence par une liaison pivot.
Le déclencheur contient des éléments de rétention permettant de contenir un ressort pré-comprimé entre le piston et le déclencheur. De préférence, le ressort représente l'unique source d'énergie de l'inserteur.
La canule est orientée selon l'orientation principale du boîtier et est maintenue, p.ex. par l'aiguille.

L'inserteur est fixé au patch grâce à des moyens de retenue disposés sur ledit patch, par exemple au moyen d'un mécanisme à baïonnette ou de clips. Le déclencheur dispose de moyens de libération du patch qui permet à l'inserteur de se désolidariser du patch une fois la canule insérée. Dans cette configuration, l'inserteur peut avantageusement être utilisé comme poignée de préhension facilitant un positionnement adéquat du patch sur la peau.

Dans une autre réalisation, l'inserteur est placé directement en contact de la peau du patient et le support de canule dispose d'une bande adhésive afin de coller le support sur la peau du patient.

Avant insertion de la canule, l'aiguille est guidée dans le boîtier pour empêcher sa rotation.
Après insertion de la canule, les éléments de butée du boîtier impartissent une rotation de l'aiguille et la contraignent à se loger dans l'espace du boîtier prévu à cet effet.

Avantageusement, l'inserteur selon l'invention comprend des éléments de sécurité qui empêchent une rotation inverse de l'aiguille une fois que celle-ci est logée dans l'espace du boîtier prévu à cet effet.

Toujours dans un contexte de sécurité, l'inserteur selon l'invention peut comporter des moyens qui empêchent l'activation du déclencheur tant que le patch n'est pas sur la peau.

Une pression sur le déclencheur permet de libérer le piston, ainsi de libérer le ressort qui va faire translater le piston jusqu'à l'insertion de la canule à travers le patch et la peau. Dans cette première phase, l'aiguille agit comme mandrin qui facilite l'insertion de la canule dans la peau.

Dans une deuxième phase, une fois la canule insérée, l'utilisateur désolidarise l'inserteur du patch à l'aide d'une nouvelle pression sur le déclencheur qui permet de libérer les moyens de retenue du patch. Le retrait de l'inserteur entraîne également le retrait de l'aiguille de la canule, cette dernière restant insérée dans le patient. Pendant cette opération, le piston continue sa course en direction de l'extrémité distale de l'inserteur. Le piston se meut car il est entraîné par les moyens de propulsion qui n'ont pas encore atteints leur position de repos. Ce mouvement du piston repousse l'inserteur dans la direction opposée et facilite le retrait de l'inserteur. Ce mouvement de piston a pour effet simultané de libérer l'aiguille des guides du boîtier et la contraignent à effectuer une rotation.

### Liste des figures

L'invention sera mieux comprise ci-après au moyen de quelques exemples illustrés.
Il va de soi que l'invention ne se limite pas à ces modes de réalisation.
Figure 1 : Ensemble des éléments composant l'inserteur.
Figures 2a et 2b : Moyens de retenue du support de l'aiguille au piston.
Figure 3 : Système déclencheur-piston armé.
Figure 4 : Inserteur positionné sur son patch, avant le déclenchement.
Figures 5a et 5b : Après avoir actionné le déclencheur, la canule et l'aiguille sont insérées.
Figures 6a et 6b : Retrait de l'inserteur après que la canule ait été insérée.
Figures 7a et 7b : Chemin parcouru par les éléments d'insertion grâce au rail de guidage.

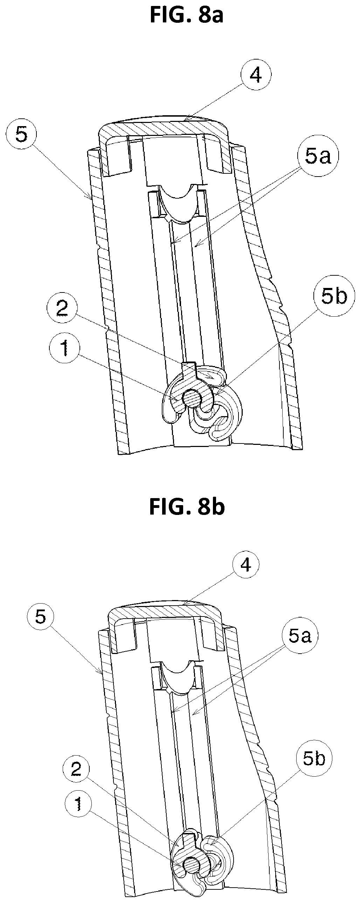

Figures 8a et 8b : Mouvement de rotation effectué par le support de l'aiguille grâce aux butées.
Figure 9 : Assemblage de l'ensemble piston, déclencheur et ressort.
Figure 10 : Ensemble piston-déclencheur avec le ressort comprimé.
Figure 11 : Assemblage du piston avec le support de l'aiguille.
Figure 12 : Ensemble des éléments d'insertion avec le dispositif de propulsion armé.
Figure 13 : Insertion de l'ensemble déclencheur-piston-aiguille-canule dans le boîtier.
Figure 14 : Fixation de l'inserteur sur le patch.
Figure 15 : Position de l'aiguille après usage.

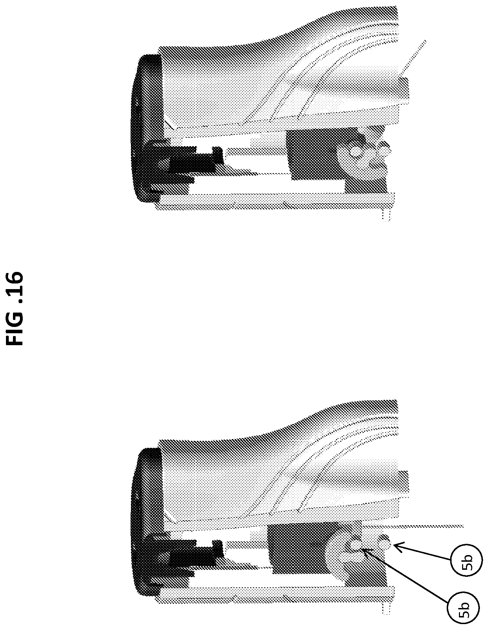

Figure 16 : Mouvement de rotation de l'aiguille.

### Description détaillée de l'invention

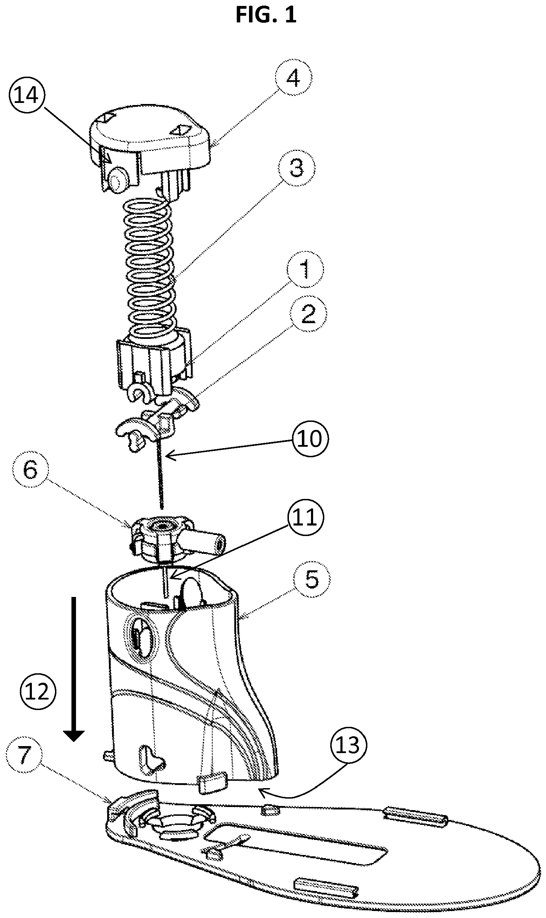

La figure 1 représente les différents composants de l'inserteur : Le piston **1,** l'aiguille surmoulée dans son support **2,** le ressort **3,** le déclencheur **4,** le boîtier **5,** la canule **11** et le patch **7.** Le déclencheur **4** comprend également au moins un bouton de sécurité **14.** La direction d'orientation principale du boitier **12** va de l'extrémité proximale de l'inserteur à l'extrémité distale de l'inserteur.

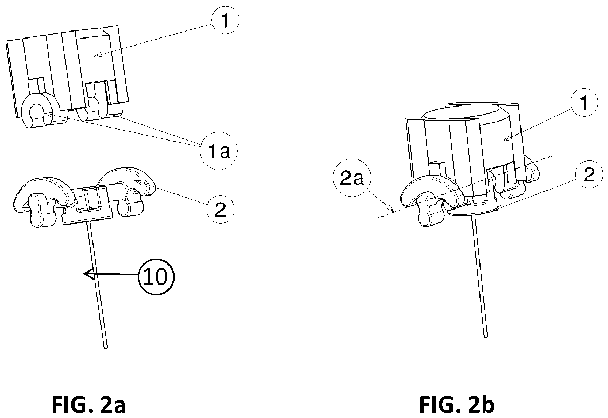

La figure 2 représente le piston **1** avec ses pinces de rétention **1a** permettant de clipser le support d'aiguille **2,** après assemblage des éléments, la rotation du support d'aiguille **2** est possible autour de l'axe **2a.**

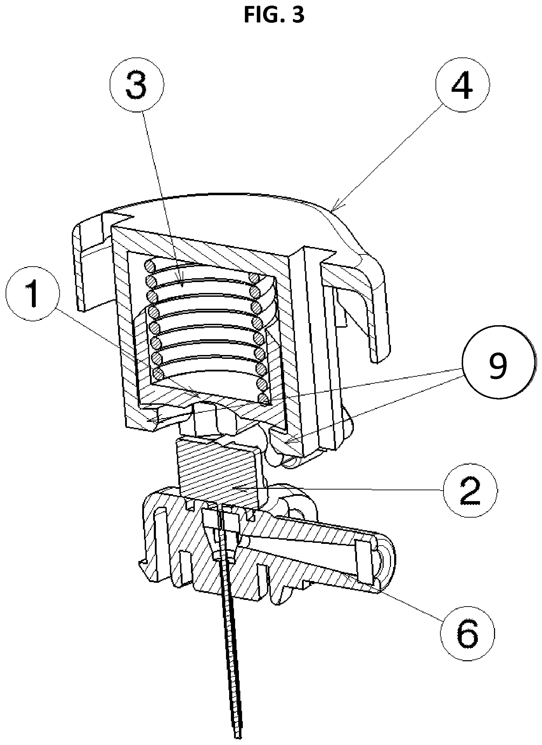

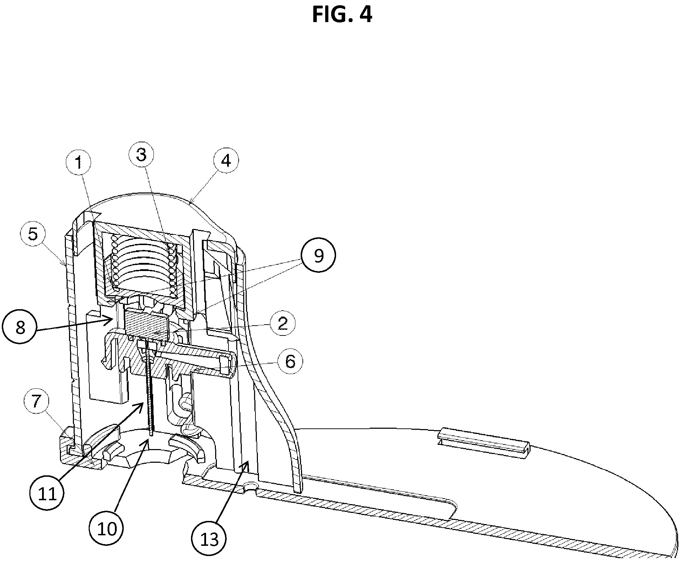

La figure 3 représente l'ensemble piston **1** - déclencheur **4** avec le ressort comprimé **3** (tel que ce sous ensemble apparaît monté dans le boîtier **5** (cf. figure 4)). Les pinces de rétention **9** permettent de maintenir le ressort **3** comprimé entre le piston inférieur **1** et le déclencheur **4.**

La figure 4 représente l'inserteur en position sur le patch **7** avant insertion, les pinces de rétention **9** permettent de garder le ressort **3** comprimé, de ce fait, l'inserteur est dans un état stable. L'aiguille **10** et la canule **11** sont en position prêt pour l'insertion.

### Etapes d'insertion et de retrait

### Figure 4

Le ressort **3** est pré-comprimé et les éléments d'insertion (**10** et **11**) sont en position pour l'insertion. Une pression sur le déclencheur **4** a pour effet d'écarter les pinces de rétention **9** grâce aux éléments de libération **8** et de libérer le piston **1.**

### Figures 5a et 5b

Une fois déclenché, une première détente du ressort **3** entraîne l'insertion de l'aiguille **10** et de la canule **11** à travers le patch **7** et la peau du patient. Le support de canule **6** vient se fixer au patch **7,** par ex. grâce à des clips. Ainsi, le support de canule **6** et le patch **7** deviennent au moins temporairement solidaire. Le piston **1,** l'aiguille **10** et son support **2** se trouvent dans une position temporaire. Dans cette position temporaire, la canule **11** est insérée et le ressort **3** reste en partie comprimé.

Une nouvelle pression sur le déclencheur **4** permet de libérer les moyens de retenue **16** grâce aux moyens de libération **15** pour désolidariser l'inserteur **5** du patch **7.**

### Figures 6a et 6b

Dans un second temps, lors du retrait de l'inserteur, le ressort **3** continue d'exercer une action sur le piston **1** qui poursuit son déplacement vers l'extrémité distale de l'inserteur. Ce mouvement induit un déplacement de l'inserteur, dans la direction opposée, qui facilite son retrait et informe l'utilisateur que la canule **11** est insérée. Lorsque l'aiguille **10** est retirée de la canule **11,** le ressort **3** entraine un dernier déplacement du piston **1** vers sa position maximale. Dans son déplacement qui l'amène à sa position maximale, le piston **1** contraint l'aiguille et son support **2** à tourner autour de l'axe **2a** (figure 2b).

### Mécanisme d'insertion et de rotation de l'aiguille

### Figures 7a et 7b

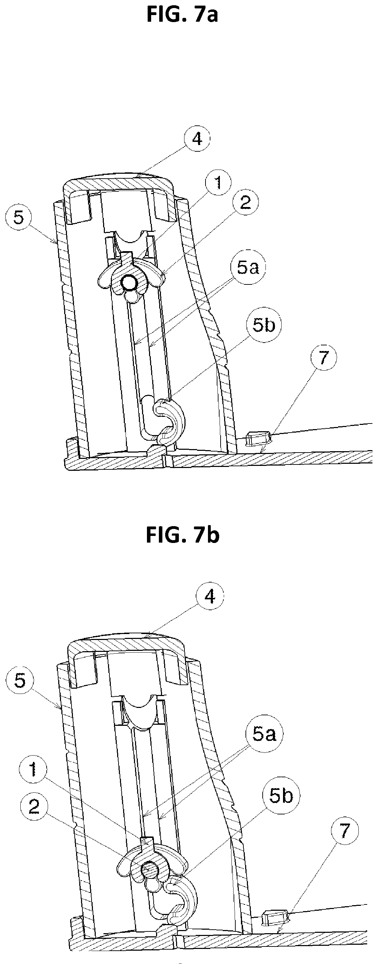

Pendant la phase d'insertion (représenté respectivement par les figures 7a à 7b), le support d'aiguille **2** est guidé dans le boîtier **5** grâce aux guides **5a** pour éviter sa rotation tout en étant maintenu par le piston **1.**

### Figures 8a et 8b

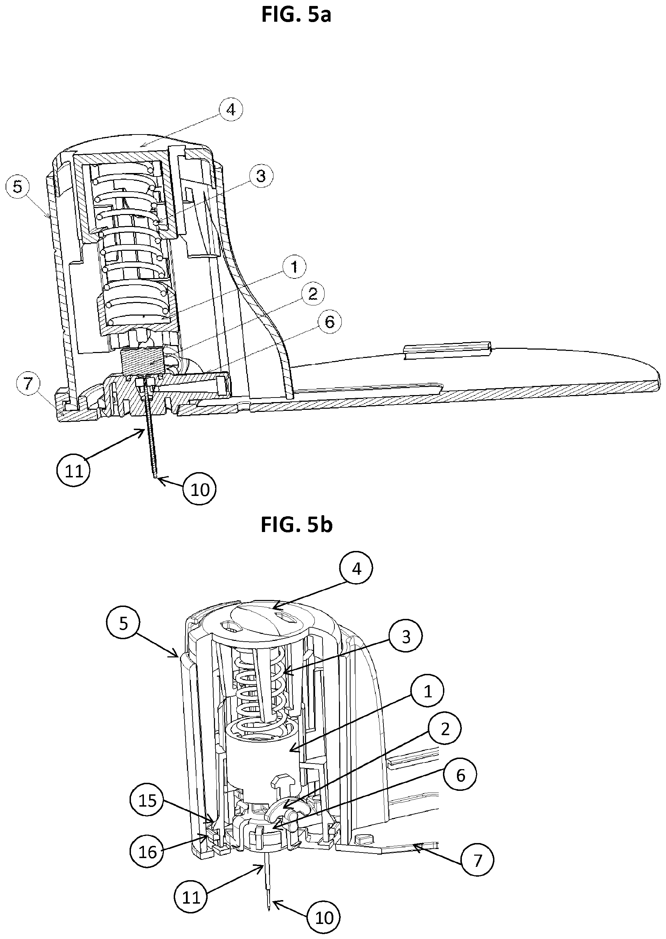

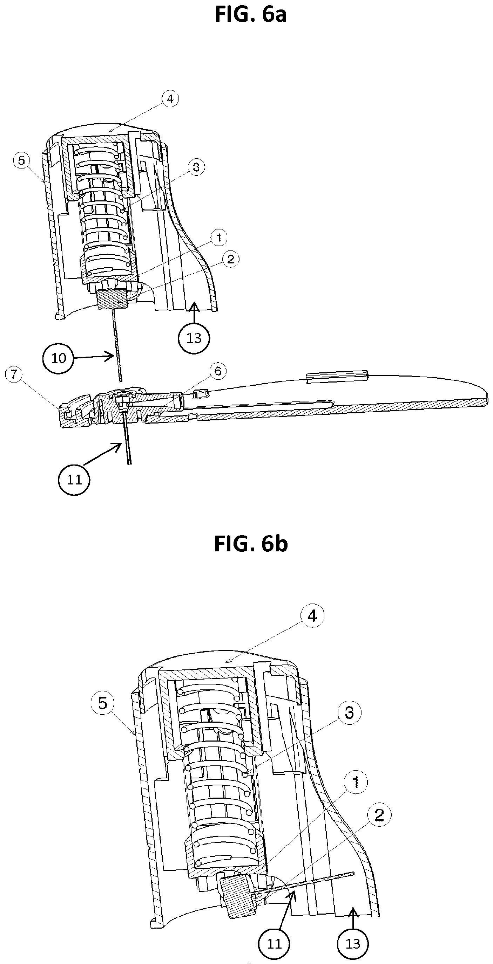

Après avoir manuellement retiré l'inserteur, l'action exercée par le ressort **3** (figure 5a) sur le piston **1** entraîne son déplacement vers l'extrémité distale du boitier **5** et repousse l'inserteur dans la direction opposée. Le piston se déplace une dernière fois pour atteindre sa position maximale et par conséquent le support d'aiguille **2** (figure 5a) va se mouvoir contre la butée **5b** du boîtier, de ce fait, par design, obliger l'aiguille **2** à tourner. L'aiguille usagée est ainsi mise à l'écart à l'intérieur de l'inserteur afin de protéger le patient ou tout autre personne manipulant ou pas l'inserteur après son utilisation. En particulier, l'aiguille se loge dans un logement **13** (figure 6a) ayant au moins pour effet de mettre en sécurité ladite aiguille.

### Figure 16

Cette figure représente le mécanisme de rotation de l'aiguille résultant du contact entre le support d'aiguille et des butées **5b** disposées dans le boîtier.

### Fixation de la canule sur le patch

La canule est conçue de sorte qu'elle soit p.ex. clipsée au patch lors de la phase d'insertion.

### Fixation momentanée du piston sur le déclencheur

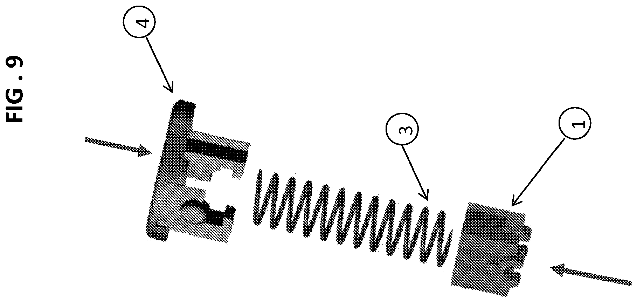

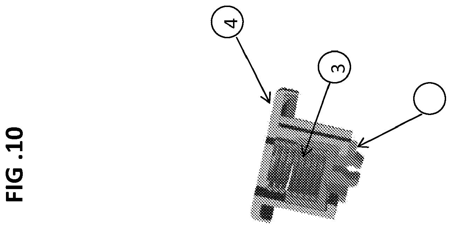

Cette étape est illustrée sur les figures 9 et 10. Une fois les pièces **4** et **1** fixées, le ressort **3** est comprimé entre celles-ci.

Cette étape lors de laquelle le ressort **3** est comprimé peut se produire avant le stockage de l'inserteur ou juste avant son utilisation.

### Fixation de la canule sur le piston

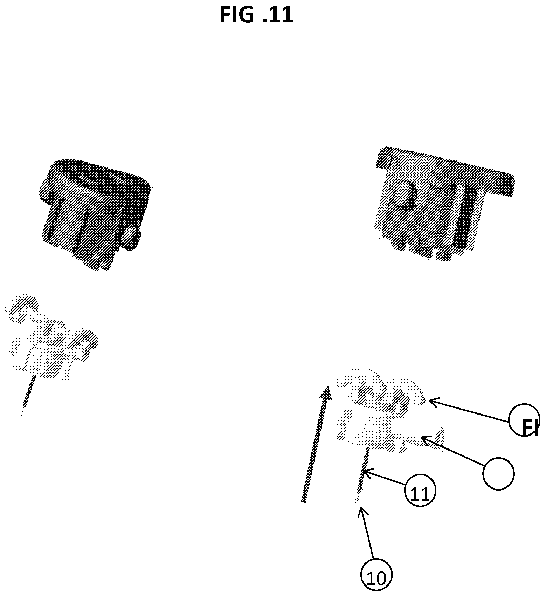

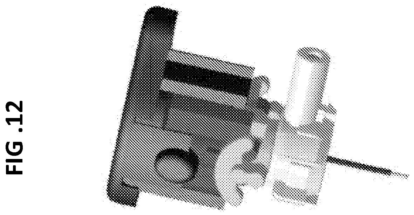

Cette étape est illustrée sur les figures 11 et 12.

### Insertion de l'ensemble déclencheur-piston-aiguille-canule dans le boîtier

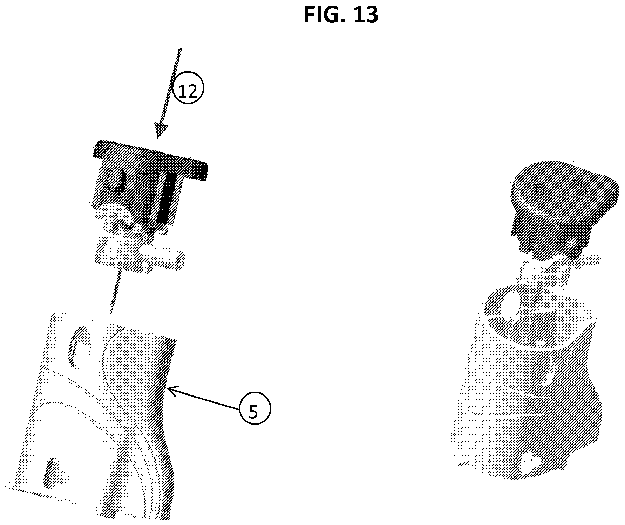

Cette insertion est illustrée sur la figure 13. A relever que l'ensemble est inséré du côté proximal de l'inserteur.
De préférence, l'aiguille et la canule sont disposées dans le boîtier **5** selon une direction parallèle à l'orientation principale **12** du boîtier **5.**
Il est cependant également possible de prévoir une configuration dans laquelle ces deux éléments sont disposés selon une direction qui forme un angle non nul par rapport à l'orientation principale **12** du boîtier **5.**

### Fixation de l'inserteur sur le patch

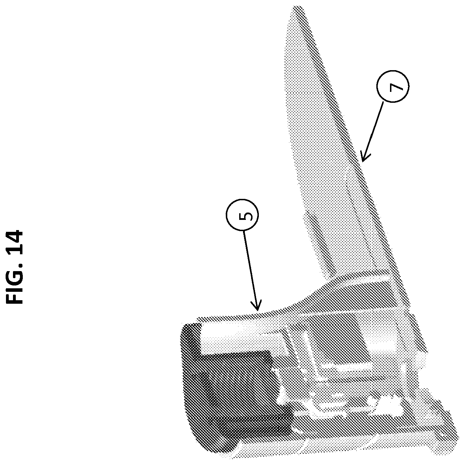

Cette dernière étape de préparation avant l'insertion de la canule est illustrée sur la figure 14. Dans cet exemple, l'inserteur est fixé sur le patch au moyen d'un mécanisme de baïonnette. Dans cette position, l'inserteur est prêt à l'emploi.

### Position de l'aiguille après usage

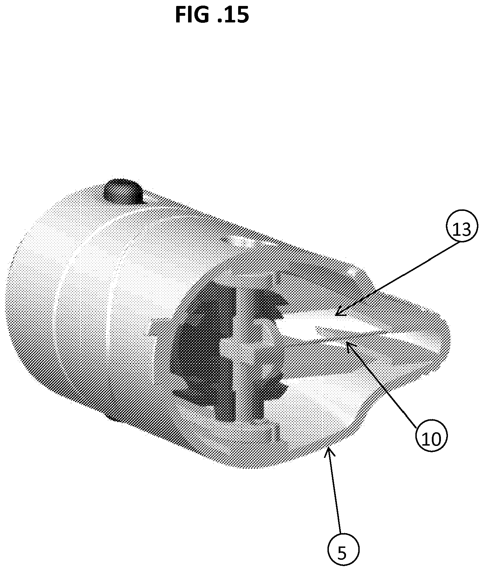

Cette étape finale est illustrée sur la figure 15.
Dans cet exemple, l'aiguille est dirigée selon une direction perpendiculaire par rapport à l'orientation principale du boîtier.

Il convient enfin de relever encore une fois que l'invention ne se limite pas aux exemples présentés dans cette demande.

Une telle canule peut notamment, mais non seulement, être utilisée pour l'injection d'insuline à un patient au moyen d'une pompe venant se fixer sur le patch qui rentre en connexion fluidique avec la canule.

### Liste des références numériques utilisées dans les figures :

**1.** Piston
**1a.** Pinces de rétention
**2.** Support d'aiguille
**2a. Axe**
**3.** Ressort
**4.** Déclencheur
**5.** Boîtier
**5a.** Guide
**5b.** Butée
**6.** Support de canule
**7.** Patch
**8.** Elément de libération
**9.** Pinces de rétention
**10**.Aiguille
**11**.Canule
**12.**Direction d'orientation principale du boîtier
**13.**Espace de logement d'aiguille
**14.**Bouton de sécurité
**15.**Elément de libération de l'inserteur
**16.**Elément de retenue du patch

## Revendications

1. Inserteur de canule (11) comprenant :
• un boîtier (5) ayant une extrémité distale destinée à venir à proximité de la peau d'un patient et une extrémité proximale opposée,
• un piston (1) fixé au moins temporairement par des moyens de retenue (9),
• des moyens d'entrainement agencés de manière à déplacer le piston (1) en direction de l'extrémité distale du boitier,
• des moyens de libération (8) destinés à libérer le piston (1); et
• une aiguille (10) fixée à un support (2) ;
le piston (1) étant monté dans le boîtier (5) de manière à ce qu'une fois libéré, il atteigne une première position; l'aiguille (10) et son support (2) étant en outre disposés de manière à pouvoir être entraînés par le piston (1) selon une direction correspondant à l'orientation principale (12) du boîtier (5), avant que le piston (1) atteigne ladite première position, **caractérisé en ce que** le piston est monté dans le boîtier (5) de manière à ce qu'il reste temporairement dans la première position et à ce qu'il atteigne et reste dans une deuxième position à l'intérieur de l'inserteur (11) lors du retrait de l'inserteur de la peau, et **en ce que** l'inserteur comprend en outre des moyens de guidage (5a) et de butée (5b) disposés de manière à ce que l'aiguille (10) et son support (2) puissent être guidés et effectuer une rotation automatique autour d'un axe perpendiculaire à ladite orientation principale (12) lors de la transition du piston entre ladite première et ladite deuxième position par l'action des moyens d'entrainement.

2. Inserteur selon la revendication 1 comprenant en outre un déclencheur et dans lequel les moyens d'entrainement sont des moyens de propulsion (3) disposés entre le piston (1) et le déclencheur (4).

3. Inserteur selon la revendication 1 dans lequel les moyens d'entrainement sont des moyens de traction disposés entre le piston (1) et l'extrémité distale de l'inserteur.

4. Inserteur selon la revendication 1 dans lequel, après ladite première position, les moyens d'entrainement exercent une force dans l'orientation principale (12) afin d'entrainer le piston (1) dans la même direction jusqu'à ladite deuxième position de manière à induire une rotation automatique de l'aiguille (10) et de son support (2).

5. Inserteur selon la revendication 1 dans lequel les moyens d'entrainement sont un ressort (3), un élastique ou une lame élastique.

6. Inserteur selon les revendications précédentes dans lequel les moyens d'entrainement sont composés d'une unique source d'énergie.

7. Inserteur selon la revendication 1 comprenant en outre un déclencheur et dans lequel les moyens de retenue (9) sont disposés sur le déclencheur.

8. Inserteur selon l'une des revendications précédentes dans lequel le boîtier comporte un espace (13) destiné à loger intégralement ladite aiguille (10) lorsqu'elle est orientée selon une direction qui diffère de ladite orientation principale (12), le dit logement agissant comme protection de l'aiguille contre toute lésion involontaire.

9. Inserteur selon l'une des revendications précédentes comprenant des éléments de butée (5b) disposés sur le boîtier (5) et sur le support d'aiguille (2), de manière à autoriser une rotation automatique de l'aiguille (10) dans ledit espace (13) lorsque le piston (1) se déplace entre ladite première position et ladite deuxième position.

10. Inserteur selon les revendications 1 à 8 dans lequel les moyens d'entrainement exercent une force suffisante sur le piston de manière à garantir le maintien de l'aiguille (10) dans son logement (13).

11. Inserteur selon l'une des revendications précédentes dans lequel le piston (1) comporte une face distale destinée à exercer exclusivement un effet de poussée sur une canule (11) et son support (6).

12. Inserteur selon l'une des revendications 1 à 10 dans lequel la canule (11) et son support (6) sont fixés au piston (1) pendant le déplacement vers l'extrémité distale du boîtier (5).

13. Inserteur selon l'une des revendications précédentes dans lequel le boîtier (5) comporte des moyens de guidage (5a) pour guider et éviter la rotation accidentelle de l'aiguille (10) et de son support (2) lors de l'insertion de la canule (11).

14. Ensemble comprenant un inserteur selon l'une des revendications 1 à 10 et une canule (11) comportant des clips ou autres moyens similaires disposés de manière à fixer la canule à un patch (7).

15. Ensemble selon l'une des revendications précédentes dans lequel le boîtier (5) est configuré de manière à agir comme organe de préhension pour faciliter le positionnement d'un patch sur la peau du patient.

## Patentansprüche

1. Kanüleneinführer (11), umfassend:
- ein Gehäuse (5) mit einem distalen Ende, das dazu bestimmt ist, in die Nähe der Haut eines Patienten zu kommen, und einem gegenüberliegenden proximalen Ende,
- einen Kolben (1), der zumindest vorübergehend durch Haltemittel (9) befestigt ist,
- Antriebsmittel, die angeordnet sind, um den Kolben (1) in Richtung des distalen Endes des Gehäuses zu verschieben,
- Freigabemittel (8), die dazu bestimmt sind, den Kolben (1) freizugeben; und
- eine Nadel (10), die an einem Träger (2) befestigt ist;
wobei der Kolben (1) in dem Gehäuse (5) derart montiert ist, dass er, wenn er freigegeben ist, eine erste Position erreicht; wobei die Nadel (10) und ihr Träger (2) ferner derart angeordnet sind, dass sie von dem Kolben (1) in eine Richtung entsprechend der Hauptausrichtung (12) des Gehäuses (5) angetrieben werden können, bevor der Kolben (1) die erste Position erreicht,
**dadurch gekennzeichnet, dass** der Kolben in dem Gehäuse (5) derart montiert ist, dass er vorübergehend in der ersten Position bleibt, und dass er eine zweite Position innerhalb des Einführers (11) beim Herausziehen des Einführers aus der Haut erreicht und in dieser bleibt, und dass der Einführer ferner Führungs- (5a) und Anschlagmittel (5b) umfasst, die derart angeordnet sind, dass die Nadel (10) und ihr Träger (2) geführt werden und eine automatische Drehung um eine Achse senkrecht auf die Hauptausrichtung (12) beim Übergang des Kolbens zwischen der ersten und der zweiten Position durch die Wirkung der Antriebsmittel ausführen können.

2. Einführer nach Anspruch 1, ferner umfassend einen Auslöser, und bei dem die Antriebsmittel Vorschubmittel (3) sind, die zwischen dem Kolben (1) und dem Auslöser (4) angeordnet sind.

3. Einführer nach Anspruch 1, bei dem die Antriebsmittel Zugmittel sind, die zwischen dem Kolben (1) und dem distalen Ende des Einführers angeordnet sind.

4. Einführer nach Anspruch 1, bei dem nach der ersten Position die Antriebsmittel eine Kraft in die Hauptausrichtung (12) ausüben, um den Kolben (1) in dieselbe Richtung bis zur zweiten Position anzutreiben, um eine automatische Drehung der Nadel (10) und ihres Trägers (2) hervorzurufen.

5. Einführer nach Anspruch 1, bei dem die Antriebsmittel eine Feder (3), ein Gummiband oder eine elastische Lamelle sind.

6. Einführer nach den vorhergehenden Ansprüchen, bei dem die Antriebsmittel von einer einzigen Energiequelle gebildet sind.

7. Einführer nach Anspruch 1, ferner umfassend einen Auslöser, und bei dem die Haltemittel (9) auf dem Auslöser angeordnet sind.

8. Einführer nach einem der vorhergehenden Ansprüche, bei dem das Gehäuse einen Raum (13) umfasst, der dazu bestimmt ist, zur Gänze die Nadel (10) aufzunehmen, wenn sie in eine Richtung ausgerichtet ist, die sich von der Hauptausrichtung (12) unterscheidet, wobei die Aufnahme als Schutz der Nadel gegen jegliche unbeabsichtigte Verletzung dient.

9. Einführer nach einem der vorhergehenden Ansprüche, umfassend Anschlagelemente (5b), die auf dem Gehäuse (5) und auf dem Nadelträger (2) angeordnet sind, um eine automatische Drehung der Nadel (10) in dem Raum (13) zu gestatten, wenn sich der Kolben (1) zwischen der ersten Position und der zweiten Position verschiebt.

10. Einführer nach den Ansprüchen 1 bis 8, bei dem die Antriebsmittel eine ausreichende Kraft auf den Kolben ausüben, um den Halt der Nadel (10) in ihrer Aufnahme (13) zu gewährleisten.

11. Einführer nach einem der vorhergehenden Ansprüche, bei dem der Kolben (1) eine distale Fläche umfasst, die dazu bestimmt ist, ausschließlich eine Schubwirkung auf eine Kanüle (11) und ihren Träger (6) auszuüben.

12. Einführer nach einem der Ansprüche 1 bis 10, bei dem die Kanüle (11) und ihr Träger (6) am Kolben (1) während der Verschiebung zum distalen Ende des Gehäuses (5) befestigt sind.

13. Einführer nach einem der vorhergehenden Ansprüche, bei dem das Gehäuse (5) Führungsmittel (5a) umfasst, um zu führen und die zufällige Drehung der Nadel (10) und ihres Trägers (2) beim Einsetzen der Kanüle (11) zu verhindern.

14. Einheit, umfassend einen Einführer nach einem der Ansprüche 1 bis 10 und eine Kanäle (11), umfassend Clips oder sonstige ähnliche Mittel, die angeordnet sind, um die Kanüle an einem Patch (7) zu befestigen.

15. Einheit nach einem der vorhergehenden Ansprüche, bei der das Gehäuse (5) eingerichtet ist, um als Greifelement zu dienen, um die Positionierung eines Patch auf der Haut des Patienten zu erleichtern.

## Claims

1. Cannula inserter (11) comprising:
• a casing (5) having a distal end intended to come into the proximity of the skin of a patient, and an opposite proximal end,
• a piston (1) at least temporarily fixed by retaining means (9),
• drive means arranged in such a way as to move the piston (1) towards the distal end of the casing,
• release means (8) intended to release the piston (1); and
• a needle (10) fixed to a support (2);
the piston (1) being mounted in the casing (5) in such a way that once released it reaches a first position; the needle (10) and its support (2) also being arranged in such a way as to be able to be driven by the piston (1) in a direction corresponding to the main orientation (12) of the casing (5), before the piston (1) reaches the said first position,
**characterized in that** the piston is mounted in the casing (5) in such a way that it remains temporarily in the first position and that it reaches and remains in a second position inside the inserter (11) when the inserter is withdrawn from the skin, and **in that** the inserter further comprises guide means (5a) and stop means (5b) positioned in such a way that the needle (10) and its support (2) can be guided and can effect an automatic rotation about an axis perpendicular to the said main orientation (12) as the piston makes the transition between the said first and the said second position through the action of the drive means.

2. Inserter according to Claim 1, further comprising a trigger and in which the drive means are propulsion means (3) arranged between the piston (1) and the trigger (4).

3. Inserter according to Claim 1, in which the drive means are traction means positioned between the piston (1) and the distal end of the inserter.

4. Inserter according to Claim 1, in which, after the said first position, the drive means apply a force in the main orientation (12) so as to drive the piston (1) in the same direction as far as the said second position so as to cause automatic rotation of the needle (10) and of its support (2).

5. Inserter according to Claim 1, in which the drive means are a spring (3), an elastic band or an elastic blade.

6. Inserter according to the preceding claims, in which the drive means are made up of a single energy source.

7. Inserter according to Claim 1 further comprising a trigger and in which the retaining means (9) are positioned on the trigger.

8. Inserter according to one of the preceding claims, in which the casing comprises a space (13) intended to fully house the said needle (10) when the latter is oriented in a direction that differs from the said main orientation (12), the said housing serving to protect the needle against any unintentional lesion.

9. Inserter according to one of the preceding claims, comprising stop elements (5b) positioned on the casing (5) and on the needle support (2) in such a way as to allow automatic rotation of the needle (10) in the said space (13) when the piston (1) moves between the said first position and the said second position.

10. Inserter according to Claims 1 to 8, in which the drive means apply enough force to the piston to ensure that the needle (10) is kept in its housing (13) .

11. Inserter according to one of the preceding claims, in which the piston (1) comprises a distal face intended to exert exclusively a thrusting effect on a cannula (11) and the support (6) thereof.

12. Inserter according to one of Claims 1 to 10, in which the cannula (11) and its support (6) are fixed to the piston (1) during the movement toward the distal end of the casing (5).

13. Inserter according to one of the preceding claims, in which the casing (5) comprises guide means (5a) for guiding and preventing accidental rotation of the needle (10) and its support (2) as the cannula (11) is being inserted.

14. Assembly comprising an inserter according to one of Claims 1 to 10 and a cannula (11) comprising clips or other similar means positioned in such a way as to attach the cannula to a patch (7).

15. Assembly according to one of the preceding claims, in which the casing (5) is configured in such a way as to act as a member for grasping so as to facilitate the positioning of a patch on the patient's skin.
